# EUROPEAN PATENT SPECIFICATION

(11) **EP 1 624 927 B1**
(45) Date of publication and mention of the grant of the patent: **05.05.2010**
(21) Application number: 04751951.7
(22) Date of filing: 12.05.2004
(51) Int. Cl.: A61N 1/36, A61B 5/04

(54) **IMPLANTABLE MEDICAL DEVICE WITH LEAD MANAGEMENT STRUCTURE**
IMPLANTIERBARE MEDIZINISCHE VORRICHTUNG MIT DRAHTMANAGEMENTEINRICHTUNG
DISPOSITIF MEDICAL IMPLANTABLE AVEC STRUCTURE DE GESTION DE CABLES

(30) Priority: 16.05.2003 US 471262 P; 20.09.2003 US 503945 P; 29.04.2004 US 835232
(43) Date of publication of application: 15.02.2006
(73) Proprietor: Medtronic, Inc., Minneapolis, MN 55432-5604 (US)
(72) Inventor: SINGHAL, Ruchika, Minneapolis, MN 55401 (US); WAHLSTRAND, Carl, D., Lino Lakes, MN 55038 (US); SKIME, Robert, M., Coon Rapids, MN 55448 (US)
(74) Representative: Hughes, Andrea Michelle
(86) International application number: PCT/US2004/014806
(87) International publication number: WO 2004/103468

(56) References cited:
- WO-A-01/28622
- WO-A-2004/043536
- US-A- 6 006 124
- US-B1- 6 427 086

## Description

The invention relates to implantable medical devices that deliver therapy to and/or monitor a patient.

Implantable medical devices (IMDs) include devices implantable in a mammalian body that sense medical parameters, monitor medical conditions, administer therapy, or any combination thereof. Typical IMDs include a variety of electrical and/or mechanical components, often including a housing that houses the components. Because the components may be fragile, the housing is usually sufficiently robust to protect the components from forces to which they would otherwise be exposed when implanted within the body. Housings may be constructed from titanium, for example. In order to avoid potentially harmful interactions between the components and bodily fluids, such as corrosion, IMD housings are typically hermetically sealed.

Large components common to most IMDs typically include a battery, a coil, and a hybrid circuit that includes digital circuits, e.g., integrated circuit chips and/or a microprocessor, and analog circuit components. IMDs may include other components as well. The components and the housing each add bulk to the IMD.

Some medical devices may be implanted in the head of a patient. For example, an IMD may be implanted under the scalp and on top of the cranium, with one or more leads deployed on the head or implanted in the brain. In many cases, the implantation is not permanent, and it may be advantageous to remove the device for reasons such as repair, maintenance, replacement, or because the patient no longer benefits from the device.

Explantation of a cranially implanted IMD includes making an incision in the scalp of a head of a patient to obtain access to the IMD, and removing the IMD.

Documents US 6 006 124 and US 6 356 792 disclose devices as described in the preamble of claim 1.

One of the risks associated with explantation is that the leads may be damaged. Typical leads can be readily damaged by a scalpel used to incise the scalp. Damage to the leads is often undesirable because removal of one IMD may be followed by implantation of another IMD, and it can be more beneficial to use leads already deployed than to deploy new leads. Accordingly, many of the embodiments of the invention are directed to protecting the leads against inadvertent damage. Some of the embodiments are directed to locating the leads so that the surgeon can plan the incision to avoid the leads, and other embodiments are directed to protecting the leads in the event the incision is made proximate to the leads.

Another risk associated with explantation is the incision may cut across the IMD itself. As a result, the IMD may be damaged, or the explantation may be hindered or complicated by a poorly placed incision. Many of the embodiments of the invention are directed to protecting the leads against inadvertent damage. Some of the embodiments are directed to locating the IMD so that the surgeon can plan an incision that will achieve the goals of the surgical procedure.

A further risk associated with explantation is that removal of the IMD may be difficult because of factors such as tissue growth proximate to the implantation site. Some of the embodiments are directed to structural features of the IMD that permit the surgeon to apply force to the IMD to dislodge it or remove it

There are additional risks associated with explantation. Incision over the top of an IMD or leads may not only damage the implanted elements, but may also adversely affect the health of the patient by, for example, damaging blood vessels, damaging nerves and increasing the risk of infection. In general, the various embodiments of the invention reduce these and other risks associated with explantation.

The invention provides an implantable medical device as defined in claim 1. In one embodiment, the invention is directed to an implantable medical device comprising at least one module that includes control electronics within a housing, a member that at least partially encapsulates the housing, and a grippable access structure coupled to the member. The device, which is configured to be implanted between a scalp and a skull of a patient, can also include a radiopaque element. The grippable access structure may be, for example, a handle, a loop or a tab.

In another embodiment, the invention presents an implantable medical device, configured to be implanted between a scalp and a skull of a patient, comprising a module that includes control electronics within a housing, member that at least partially encapsulates the housing, and a radiopaque element. The radiopaque element may be a part of the housing itself, for example, or may be a radiopaque marker.

In a further embodiment, the invention is directed to an implantable medical device configured to be implanted between a scalp and a skull of a patient. The device includes at least one module that includes control electronics within a housing and a lead management structure. The lead management structure is configured to receive and protect bodies of leads coupled to the implantable medical device. The lead management structure may comprise a groove around the periphery of the device, for example.

In an additional embodiment, the invention presents burr hole cap, comprising a lead management structure configured to receive and protect coiled bodies of leads passing through the burr hole cap. The lead management structure may comprise a groove in one of the members of the burr hole cap.

In another embodiment, the invention is directed to an implantable medical device comprising a pouch made of cut-resistant material. The pouch is sized to receive a coil of a lead implanted in a body, and may include a radiopaque element.

The invention may be used in a method comprising receiving an image of a patient, determining a location of an implantable medical device implanted between a scalp and a skull of the patient based on the image, and making an incision in the scalp based upon the determination. The method can optionally include gripping a grippable access structure of the implantable medical device and applying force to the implantable medical device via the grippable access structure.

Preferred embodiments will now be described, by way of example only, with reference to the drawings.

FIG. 1 is a conceptual diagram illustrating deployment of a low-profile IMD under the scalp of a patient.

FIG. 2 is a plan diagram of the top of a head of a patient, illustrating an exemplary implantation of a low-profile IMD.

FIG. 3 is a conceptual imaging diagram of the top of a head of a patient, illustrating an exemplary technique for identifying the location of an implanted low-profile IMD.

FIG. 4 is a plan diagram of one embodiment of a low-profile IMD that includes a grippable access structure in the form of a loop.

FIG. 5 is a plan diagram of another embodiment of a low-profile IMD that includes a grippable access structure in the form of a tab.

FIG. 6 is a plan diagram of the top of a head of a patient, illustrating an exemplary implantation of a low-profile IMD with a tethered interconnect.

FIG. 7 is a perspective view of an embodiment of a low-profile IMD that includes a lead management structure.

FIG. 8 is a perspective view of an embodiment of a burr hole cap that includes a lead management structure.

FIG. 9 is a perspective view of an embodiment of a protective lead pouch.

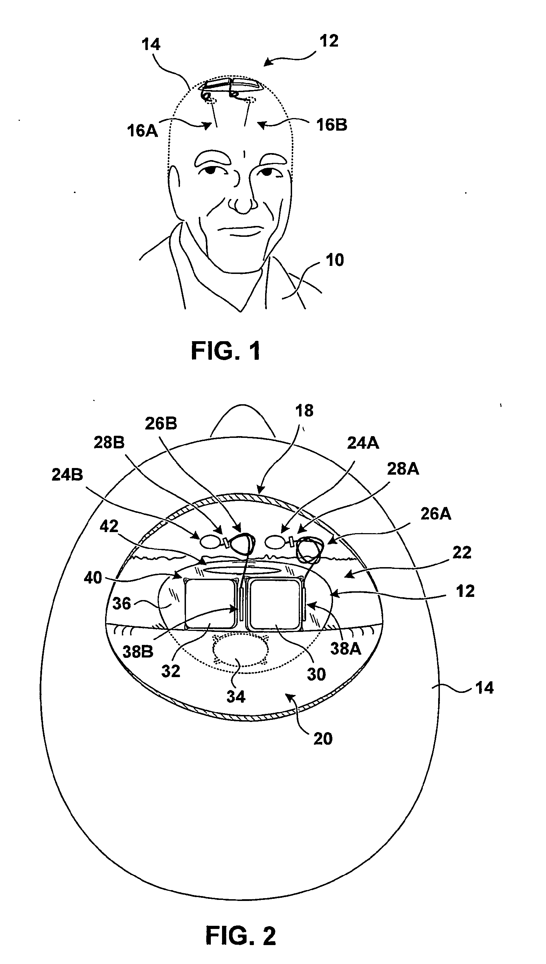

FIG. 1 shows a patient 10 with a low-profile IMD 12 deployed beneath his scalp 14. In FIG. 1, IMD 12 is a neurostimulator that provides deep brain stimulation via leads 16A, 16B deployed in the brain of patient 10. In the example of FIG. 1, IMD 12 is deployed in proximity to site of stimulation therapy. IMD 12 may be used to treat any nervous system disorder including, but not limited to, epilepsy, pain, psychological disorders including mood and anxiety disorders, movement disorders (MVD) such as, but not limited to, essential tremor and Parkinson's disease and neurodegenerative disorders.

Although IMD 12 is depicted as a neurostimulator, the invention is not limited to applications in which the IMD is a neurostimulator. The invention may be employed with IMDs that perform any monitoring or therapeutic functions. The invention is not limited to IMD that include leads deployed in the brain, but may also be employed with leads deployed anywhere in the head or neck including, for example, leads deployed on or near the surface of the skull, leads deployed beneath the skull such as near or on the dura mater, leads placed adjacent cranial or other nerves in the neck or head, or leads placed directly on the surface of the brain. Nor is the invention limited to IMDs that are coupled to electrodes. The invention may be employed with low-profile IMDs coupled to any sensing or therapeutic elements, such as temperature sensors or motion sensors. The invention may also be employed with different types of IMDs including, but not limited to, IMDs operating in an open loop mode (also referred to as non-responsive operation), IMDs operating in a closed loop mode (also referred to as responsive), and IMDs for providing monitoring and/or warning.

In the example of FIG. 1, IMD 12 is deployed beneath scalp 14 of patient 10, but on top of the cranium of patient 10. The invention may be applied to other types of implantation as well, such as implantation of IMD 12 in a trough cut into the cranium of patient 10.

A surgeon may implant IMD 12 using any surgical technique. In a typical implantation, the surgeon makes an incision through the scalp 14 of patient 10, and pulls back the resulting flap of skin to expose the desired area of the cranium. The incision may be a "C-flap" incision, for example. The surgeon drills holes, called "burr holes," in the cranium and deploys leads 16 through the burr holes into the brain.

The surgeon typically places caps, called "burr hole caps," over the burr holes. Before connecting leads 16 to IMD 12, the surgeon typically "manages" the leads. Lead management includes arranging the excess length of leads 16 using techniques such as coiling and anchoring with anchoring plates. In a typical implantation, the surgeon arranges the leads to provide some slack to reduce the risk of lead migration. Lead management also reduces the risk that the leads will be accidentally damaged during explantation, as described below.

The surgeon implants IMD 12 between scalp 14 and the skull. In one surgical procedure, the surgeon uses a tool to form a pocket beneath the scalp proximate to the burr holes, and positions IMD 12 in the pocket. The surgeon may fix IMD 12 to the cranium using an attachment mechanism such as bone screws. The surgeon closes the skin flap over IMD 12, and then staples or sutures the incision.

At a later date, it may be necessary to remove IMD 12 from patient 10. Explantation involves considerations that are distinct from implantation. For example, the surgeon may desire to remove IMD 12 but may desire to keep leads 16 deployed as they are. In addition, the surgeon may desire to recover IMD 12 in an undamaged condition. It may also be possible that the implanting surgeon and the explanting surgeon are different people, and the explanting surgeon may be unaware of what implantation and lead management techniques were used by the implanting surgeon. Because of considerations such as these, the explanting surgeon plans the surgery to avoid accidentally damaging the leads or the implanted device when making an incision.

FIG. 2 illustrates a procedure for explantation of IMD 12 shown in FIG. 1. FIG. 2 is a diagram showing the top of the head of patient 10. Patient 10 may be under local anesthetic. The surgeon begins explantation by making an incision such as C-flap incision 18 in scalp 14. In general, the surgeon has discretion concerning the making of an incision based upon the circumstances of each individual patient. Accordingly, the incision need not be a C-flap incision as shown in FIG. 2, but may include a straight incision or an S-shaped incision, for example. The incision chosen by the surgeon may be a function of the location of IMD 12, the location of the leads, or other factors. As shown in FIG. 2, the surgeon draws scalp flap 20 away to expose the portion of the patient's skull 22 beneath scalp flap 20, and to expose at least a portion of IMD 12.

In the example shown in FIG. 2, patient 10 has leads 16A and 16B deployed in the brain through burr holes 24A and 24B. A portion of the bodies of leads 16A and 16B, identified with reference numerals 26A and 26B, is deployed outside of the brain on the surface of skull 22. The burr holes may be sealed with burr hole caps, with leads 26A and 26B passing therethrough. Leads 26A and 26B are depicted as coiled and are anchored by anchoring plates 28A and 28B. Leads 26A and 26B are coupled to IMD 12.

In FIG. 2, IMD 12 is a low-profile device, allowing it to be implanted between scalp 14 and skull 22, with little discomfort or adverse cosmetic consequences to patient 10. In addition, low-profile IMD 12 can have the advantages of reducing skin erosion and infection. IMD 12 comprises one or more modules that carry out the various functions of IMD 12. As shown in FIG. 2, IMD 12 includes at least three modules: a control module 30, a power supply module 32 and a recharge module 34. One or more of modules 30, 32, 34 includes a housing that can carry out a variety of functions, including encasing the components of the modules, sealing the modules against contamination, electrically isolating electrical components, and the like. In some embodiments of the invention, at least one of the modules comprises a radiopaque material. The modules are coupled to member 36, which may be made of a soft, biocompatible material. Member 36 at least partially encapsulates one or more housings of modules 30, 32, 34, and generally serves as a smooth interface between the modules and the body tissue. Leads 26A and 26B are coupled to IMD 12 at lead connectors 38A and 38B. IMD 12 may be anchored with an anchoring mechanism such as a metallic tab 40 that includes an opening for receiving a bone screw.

In general, member 36 integrates modules 30, 32 and 34 into a desired form factor, but, where flexible, allows relative intermodule motion. In some embodiments, member 36 incorporates mechanical features to restrict intermodule motion to certain directions or within certain ranges. Member 36 may be made from silicone, and is some embodiments may be made from two or more materials of differing flexibility, such as silicone and a polyurethane. An exemplary polyurethane for this purpose is Tecothane®, which is commercially available from Hermedics Polymer Products, Wilmington, MA. Member 36 may also be referred to as an "overmold," but use of the term "overmold" herein is not intended to limit the invention to embodiments in which member 36 is a molded structure. Member 36 may be a molded structure, or may be a structure formed by any process.

The invention is not limited to the particular IMD depicted in FIG. 2, but includes a number of embodiments, some of which are described in more detail below.

In FIG. 2, it is assumed that the surgeon has successfully made incision 18, avoiding leads 26 and IMD 12. The surgeon may also have successfully removed bone screws that anchored IMD 12 to skull 22. The surgeon can decouple leads 26A and 26B from lead connectors 38A and 38B by hand or with a tool. In many cases, however, IMD 12 does not easily separate itself from the site of implantation, and the surgeon applies force to remove IMD 12. Fibrous tissue growth proximate to the implantation site, for example, may resist the efforts of the surgeon to remove IMD 12.

IMD 12 includes a grippable access structure 42 that aids in explantation. In FIG. 2, grippable access structure 42 is a small handle or handle-like formation in or otherwise coupled to member 36 that can be gripped with a hand or an instrument, so that the surgeon may apply force to remove IMD 12. A surgeon presented with IMD 12 as shown in FIG. 2, for example, can grip IMD 12 at handle 42 with a forceps, and apply force to pull or twist IMD 12.

The invention is not limited to the grippable access structure shown in FIG. 2. Other exemplary embodiments of grippable access structures will be described below. Some embodiments of grippable access structures have the advantage that they give more implantation and explantation options to the surgeon. In particular, the surgeon can plan an explantation procedure in which the incision is close to the grippable access structure, but safely away from the IMD and the leads.

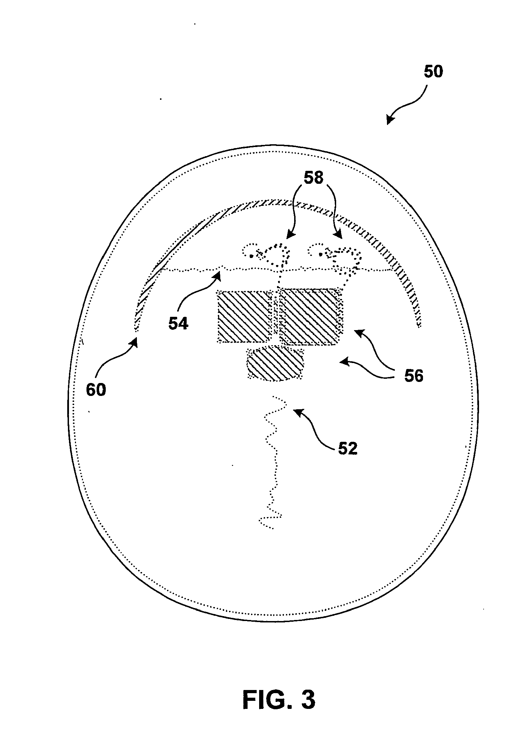

FIG. 3 is a conceptual imaging diagram of the top of a head of a patient, illustrating an exemplary technique for identifying the location of an implanted low-profile IMD. Before explanting the implanted device, the surgeon should know where the device is. Accordingly, the surgeon may direct that patient 10 be imaged using one or more medical imaging techniques such as X-ray, magnetic resonance imaging (MRI), CT-scan or fluoroscopy.

Some of the imaging techniques employ electromagnetic radiation. FIG. 3 represents an image 50 obtained with radiation, such as an X-ray. The image may include images of features or landmarks 52, 54 of the skull, which assist in locating the implanted device. In addition, FIG. 3 shows images of modules 56 of the implanted device. Images of modules 56 appear in contrast to most of the balance of image 50. Modules 56 appear because the housings include a radiopaque material that causes the modules to stand out in image 50. In the exemplary illustration of FIG. 3, the member, being made of a non-radiopaque material such as silicone, does not appear in image 50.

In some embodiments of the invention, however, the member includes one or more radiopaque markers, so that the location of the member can be identified as well. The invention supports any of several techniques for including one or more radiopaque markers in the member, such as outlining the member with radiopaque wire and loading the member with radiopaque powders or fibers.

In FIG. 3, the image of leads 58 is visible as well, as the leads may include radiopaque markers. In addition, image 50 includes a radiopaque incision mark 60, which may have been created by the surgeon who implanted the device. The surgeon can use a radiopaque marker to make radiopaque incision mark 60 on the skull of the patient during the implantation procedure. In some cases, radiopaque incision mark 60 can assist the surgeon in locating the IMD and leads by providing a reference on the skull itself. In addition to imaging as shown in FIG. 3, the surgeon could palpate for the IMD and could use the implantation incision scar as a reference. Radiopaque incision mark 60 may show the surgeon whether the implantation incision scar is proximate to its original site, or whether the implantation incision scar has migrated anteriorly or posteriorly. The surgeon can correct for scar migration, thereby reducing the risk of making an incision that cuts across the IMD. In addition, the surgeon can reduce the risk of making an incision that inadvertently cuts across the leads, which may be difficult to locate by palpation.

In general, the explanting surgeon takes one or more images of the patient, and uses the images to determine the location of the implanted device and the leads. In particular, the surgeon uses the image to learn about the size and configuration of the implanted device, and the lead management techniques that have been employed. The surgeon may also take into consideration the site of an incision used during the implantation procedure.

Using this information, the surgeon plans an incision strategy. The incision strategy takes into account the safety and effectiveness of a given incision, based upon the information obtained from the images. The surgeon implements the incision strategy in the operating room and removes the implanted device.

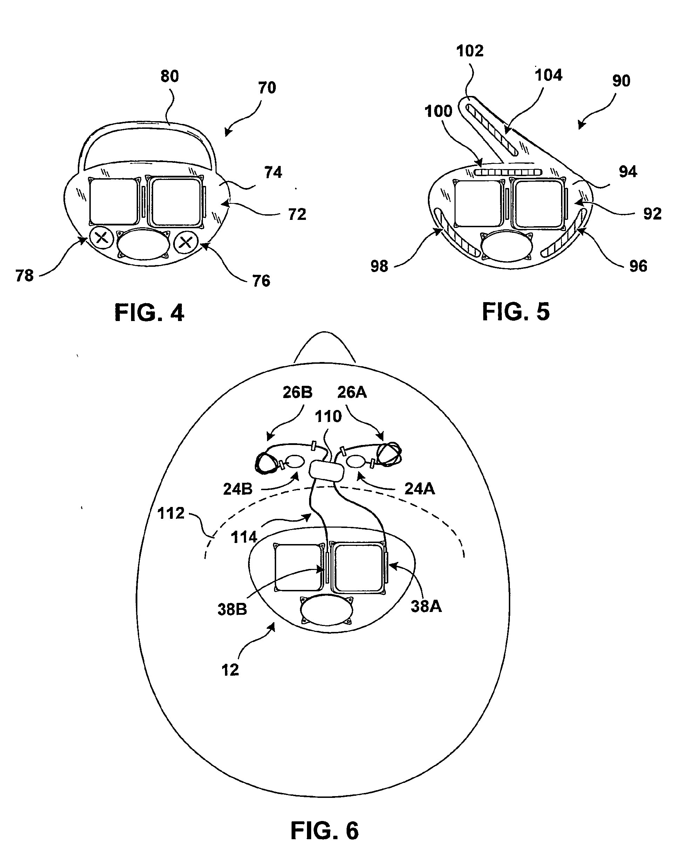

FIG. 4 shows an alternate exemplary embodiment of the invention. IMD 70 is a low-profile IMD that includes one or more modules 72 with housings that are at least partially encapsulated by a member 74. In addition, radiopaque markers 76, 78 are coupled to member 74. Markers 76, 78, which appear more plainly on an image than member 74, can assist the surgeon in locating the position of the member. Markers 76, 78 may include additional information about member 74, such as a model number, that would assist the surgeon in identifying the shape and dimensions of member 74. Markers 76, 78 may be affixed to exterior of member 74 or may be embedded in member 74.

In addition, IMD 70 includes a grippable access structure 80 coupled to member 74, in the form of a loop. Loop 80, like handle 42 in FIG. 2, can be formed integral with the member or may be mechanically coupled to the member. Loop 80 can be dimensioned such that a surgeon may grip loop 80 with an instrument such as a forceps, or the surgeon has the option to grip loop 80 with her fingers. Loop 80 may include a wire or other radiopaque element (not shown) that would make loop 80 visible during imaging.

FIG. 5 illustrates another exemplary embodiment of the invention. IMD 90 is a low-profile IMD that includes one or more modules 92 with housings that are at least partially encapsulated by a member 94. In addition, radiopaque markers 96, 98, 100 are coupled to member 94, and can assist the surgeon in locating the position of member 94 in an image. In particular, radiopaque markers 96, 98, 100 assist the surgeon in identifying the edges of IMD 90. Radiopaque markers 96, 98, 100 may be affixed to exterior of member 94 or may be embedded in member 94, such as by loading radiopaque powders or fibers in member 94.

In addition, IMD 90 includes a grippable access structure 102 coupled to member 94, in the form of a tab. Like loop 80 in FIG. 4, tab 102 can be formed integral with the member or may be mechanically coupled to the member, and can be dimensioned to give the surgeon flexibility to grip the structure by hand or with an instrument. In the embodiment shown in FIG. 5, tab 102 includes a radiopaque marker 104 that would make tab 102 visible during imaging.

FIG. 6 shows a further exemplary embodiment of the invention in an explantation procedure. In particular, FIG. 6 demonstrates a technique for lead management that may be advantageous during explantation.

FIG. 6 shows the top of the head of the patient, with the scalp being invisible for clarity. As in FIG. 2, leads 26A and 26B are coiled proximate to burr holes 24A and 24B, and IMD 12 is implanted nearby. In FIG. 6, leads 26A and 26B are coupled to IMD 12 via tethered interconnect module 110. In the embodiment shown in FIG. 6, tethered interconnect module 110 couples to the lead connectors 38A and 38B of IMD 12 and leads 26A and 26B, and is interposed between the lead connectors and the leads. With tethered interconnect module 110, the surgeon has more options for coupling leads 26A and 26B to IMD 12. The surgeon may elect, for example, to deploy leads 26A and 26B so as to create a substantial space between the leads and IMD 12.

During explantation, an incision 112 can cause damage to the interconnecting leads 114 of tethered interconnect module 110. Even so, the integrity of leads 26A and 26B is preserved. In other words, tethered interconnect module 110 can be sacrificed during explantation to avoid damage to IMD 12 and leads 26A and 26B by the incision. Once tethered interconnect module 110 is decoupled from IMD 12 and from leads 26A and 26B, the surgeon can remove IMD 12 without disturbing from leads 26A and 26B.

Tethered interconnect module 110 may include a radiopaque material that enhances its visibility during imaging. In addition, tethered interconnect module 110 may include one or more anchoring structures (not shown) that hold tethered interconnect module 110 in position. The configuration of tethered interconnect module 110 shown in FIG. 6 is exemplary, and the invention is not limited to the particular configuration shown.

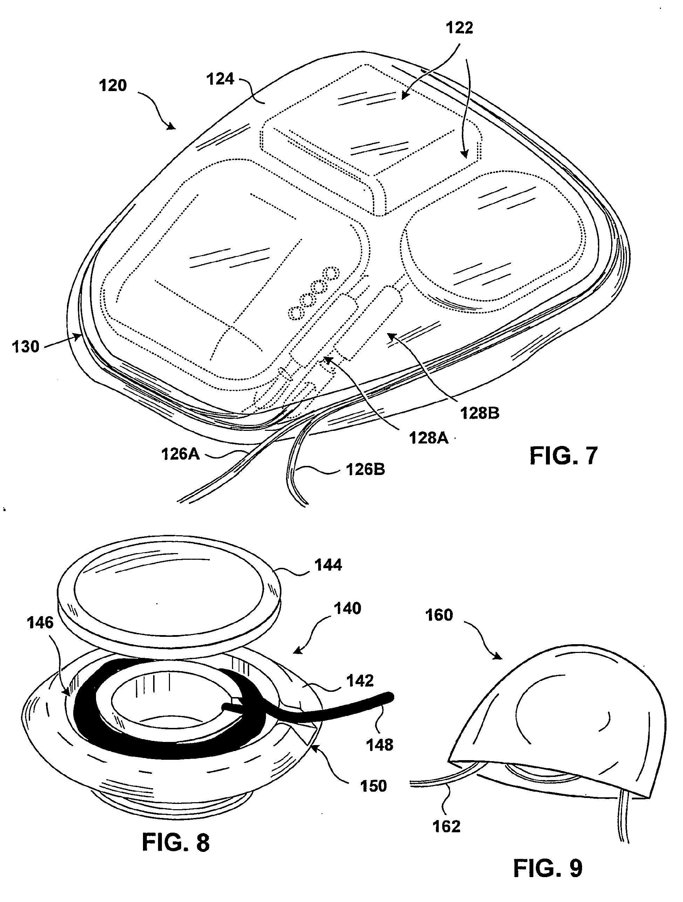

FIG. 7 is a perspective view of an embodiment of a low-profile IMD 120 that includes a lead management structure. IMD 120 includes one or more modules 122 within housings and a member that at least partially encapsulates the housings. IMD 120 is configured to be implanted between a scalp and a skull of a patient.

Leads 126A and 126B are coupled to lead connectors 128A and 128B. Leads 126A and 126B are deployed around IMD 120 in a lead management structure. A lead management structure is a structure in IMD 120 that is configured to receive and protect the bodies of leads that are coupled to the IMD. In particular, a lead management structure is a structure that is configured to receive and protect the bodies of the leads as opposed to the terminals of the leads. Lead management structures include, but are not limited to, structures that route, fixate or anchor the lead bodies. Examples of a lead management structure include a groove or a cavity that receives a lead body.

One of the practical problems associated with the leads is that the leads can be difficult to manage. The leads can twist, bend, slide and otherwise move. The propensity of leads to move can be inconvenience during implantation, and can also be a problem during explantation. If the leads move after implantation, there is an increased risk of damage to leads during explantation.

In FIG. 7, the lead management structure is a groove 130 formed in member 124, and leads 126A and 126B are wrapped around IMD 120 in groove 130. The dimensions of the groove may a function of the length of the leads and the dimensions of IMD. The placement of groove 130 around the periphery of IMD 120 is for illustrative purposes, and the invention is not limited to the particular lead management structure shown in FIG. 7.

The lead management structure need not be formed in member 124. In some embodiments, the lead management structure can be constructed of a separate material, such as a protective material that would resist damage in the event the incision should cut across IMD 120. Cut-resistant materials include, but are not limited to, metals and materials including embedded wire or polymer meshes. Furthermore, the lead management structure need not be located around the periphery as shown in FIG. 7, but in some embodiments can be located underneath member 124 and modules 122. Lead management structures can not only direct lead bodies around IMD 120, but can direct the lead bodies over or under IMD 120.

The lead management structure offers several possible benefits. First, it can protect the leads from damage in circumstances in which the incision cuts across the IMD. Second, it can in some circumstances offer a more efficient lead management option than coiling as illustrated in FIGS. 2, 3 and 6. Third, if the leads include radiopaque materials, an image of the leads can show not only the position of the leads, but also the position of the IMD.

FIG. 8 is a perspective view of an embodiment of a burr hole cap 140 that includes a lead management structure. Burr hole cap 140 comprises a ring member 142 and a cover member 144 that couples to ring member 142 by a coupling mechanism (not shown). Burr hole cap 140 is configured to close a burr hole in a bony structure such as a skull, while allowing a lead to pass through.

Ring member 142 includes a lead management structure. The lead management structure is groove 146, which receives lead 148. The implanting surgeon can coil lead 148 inside groove 146, and draw lead through exit 150, before coupling cover member 144 to ring member 142. Ring member 142, cover member 144 or both can be constructed from a protective material that would resist damage in the event the incision should cut across burr hole cover 140.

The lead management technique illustrated in FIG. 8 can protect the lead from damage in circumstances in which the incision is close to the burr holes. Burr hole cap 140 can, in some circumstances, offer a more efficient lead management option than coiling outside of the burr hole cap. The configuration of the burr hole cap and the lead management structure are for illustrative purposes, and the invention is not limited to the burr hole cap or lead management structure shown in FIG. 8. For example, the invention includes burr hole caps that include a lead management structure that supports winding of a lead around the exterior of the burr hole cap.

FIG. 9 is a perspective view of a protective pouch 160 that can be used in lead management. Pouch 160 is sized to slip over coils of lead 162 and protect the coils from accidental damage. Pouch 160 can be constructed of a cut-resistant protective material and may also include a radiopaque material that enhances visibility of pouch 160 during imaging. Pouch 160 may be constructed of any of a number of biocompatible materials, such as silicone, and may further incorporate cut-resistant materials. Cut-resistant materials include, but are not limited to, metals and materials including an embedded metallic wire mesh, embedded threads, or a polymer mesh such as a Dacron mesh.

The invention is not limited to the particular embodiment of the pouch shown in FIG. 9. The invention encompasses, for example, pouches that are configured to hold more than one lead, pouches that have anchoring structures, and pouches that include closing structures that reduce the risk that the pouch will disengage from the coiled lead.

Although the invention has been described in connection with explantation of a device implanted on the head, the invention is not limited to the area of the head. A low-profile IMD such as the devices described herein may be implanted anywhere in the body. Implantation and explantation techniques may be similar to techniques for explantation and implantation under the scalp. In particular, the surgeon may make an incision in the skin of a patient. The surgeon may retract the incision to expose a bone, muscle or other anatomical structure. The surgeon may wish to avoid damage to the IMD or the leads, and may wish to remove the IMD without disturbing the leads.

The invention supports implantation of an IMD that performs any of several functions. The invention supports explantation of IMDs that provide monitoring, IMDs that administer therapy, and IMDs that do both. The invention is not limited to any particular number of modules or to any particular functionality.

## Claims

1. An implantable medical device comprising:
at least one module (30, 32, 34) that includes control electronics within a housing; and **characterised by**
a member (36, 124) that at least partially encapsulates the housing; and
a lead management structure (130) formed in or on the member and configured to receive and protect a body of a lead that is coupled to the implantable medical device,
wherein the implantable medical device is configured to be implanted between a scalp and a skull of a patient.

2. The device of claim 1, wherein the lead management structure comprises a groove (130) formed in the member.

3. The device of claim 1, wherein the member defines a periphery and wherein the lead management structure comprises a groove around the periphery of the member.

4. The device of any preceding claim, wherein the implantable medical device includes an implantable neurostimulator.

5. The device of claim 1, wherein the lead management structure is constructed of a separate material from the member.

## Patentansprüche

1. Implantierbare medizinische Vorrichtung mit:
wenigstens einem Modul (30, 32, 34), das eine Steuerelektronik in einem Gehäuse enthält; und **gekennzeichnet durch**
ein Element (36, 124), das wenigstens teilweise das Gehäuse einschließt bzw. -kapselt; und
eine Leitungsverwaltungsstruktur (130), die in oder auf dem Element ausgebildet und dafür eingerichtet ist, einen Körper einer Leitung, welche mit der implantierbaren medizinischen Vorrichtung verbunden ist, aufzunehmen und zu schützen,
wobei die implantierbare medizinische Vorrichtung dafür eingerichtet ist, zwischen einer Kopfhaut und einem Schädel eines Patienten implantiert zu werden.

2. Vorrichtung nach Anspruch 1, bei der die Leitungsverwaltungsstruktur eine in dem Element ausgebildete Kerbe bzw. Rille (130) aufweist.

3. Vorrichtung nach Anspruch 1, bei der das Element eine Peripherie definiert, und bei der die Leitungsverwaltungsstruktur eine Rille um die Peripherie des Elements aufweist.

4. Vorrichtung nach einem der vorstehenden Ansprüche, bei der die implantierbare medizinische Vorrichtung einen implantierbaren Neurostimulator beinhaltet.

5. Vorrichtung nach Anspruch 1, bei der die Leitungsverwaltungsstruktur aus einem zu dem Element unterschiedlichen Material hergestellt ist.

## Revendications

1. Dispositif médical implantable comportant :
au moins un module (30, 32, 34) qui inclut une partie électronique de commande dans un boîtier ; et **caractérisé par**
un élément (36, 124) qui encapsule au moins partiellement le boîtier; et
une structure de gestion de dérivation (130) formée dans l'élément ou sur celui-ci et configurée pour recevoir et
protéger un corps d'une dérivation qui est couplée au dispositif médical implantable,
dans lequel le dispositif médical implantable est configuré pour être implanté entre le cuir chevelu et le crâne d'un patient.

2. Dispositif selon la revendication 1, dans lequel la structure de gestion de dérivation comporte une rainure (130) formée dans l'élément.

3. Dispositif selon la revendication 1, dans lequel l'élément défini une périphérie et dans lequel la structure de gestion de dérivation comporte une rainure autour de la périphérie de l'élément.

4. Dispositif selon l'une quelconque des revendications précédentes, dans lequel le dispositif médical implantable inclut un neurostimulateur implantable.

5. Dispositif selon la revendication 1, dans lequel la structure de gestion de dérivation est constituée d'un matériau séparé de l'élément.
